# EUROPEAN PATENT APPLICATION

(11) **EP 2 801 357 A1**
(43) Date of publication of application: **12.11.2014**
(21) Application number: 13167229.7
(22) Date of filing: 10.05.2013
(51) Int. Cl.: A61K 31/235, A61K 31/60, A61P 3/10, A61P 3/04, A61P 3/00, A61P 25/16, A61P 25/28

(54) **Carboxylated stilbenes for activating AMPK and sirtuins**

(71) Applicant: IMD Natural Solutions GmbH, 44227 Dortmund (DE)
(72) Inventor: Bitzer, Jens, 44225 Dortmund (DE); Küper, Thomas, 44137 Dortmund (DE); Wabnitz, Philipp, 40237 Düsseldorf (DE)
(74) Representative: Habermann, Hruschka & Schnabel

(57) **Abstract**

The present invention relates to the use of certain carboxylated stilbene derivatives in pharmaceutical and medical applications, in particular prevention and/or treatment of metabolic disorders and neurodegenerative diseases, as well as to pharmaceutical and medical compositions containing such stilbene derivatives.

## Description

The present invention relates to pharmaceutical compositions comprising carboxylated stilbene derivatives and their use for prevention and/or treatment of medical conditions, in particular prevention and/or treatment of metabolic disorders and neurodegenerative diseases.

There is an increasing interest in stilbenoids such as trans-resveratrol for nutraceutical, cosmetic, and pharmaceutical uses. Amongst other effects, trans-resveratrol and related hydrostilbenes are sirtuin agonists (US-A-2012/0172340) and activate AMPK (Baur (2010) Biochim. Biophys. Acta 1804(8), 1626-1634).

Hashimoto et al. (1988) Phytochemistry 27, 109-113, disclose stilbene and dihydrostilbene derivatives obtained by semi-synthetic preparation from *Hydrangea* extracts.

The technical problem underlying the present invention is to provide improved pharmacological formulations containing improved stilbenoid compounds with significantly higher biological efficacy, inspired by nature.

The solution to the above technical problem is provided by the embodiments of the present invention as described herein and in the claims.

In particular, the present invention provides a compound of general formula (I) for use as a medicament, preferably by systemic application in humans, 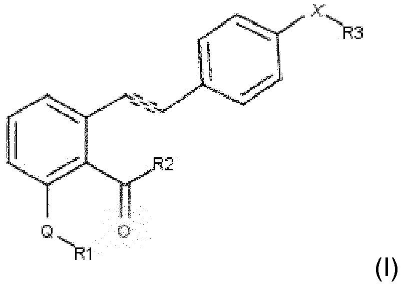 wherein 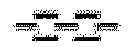 is a single or double bond;
- R¹: is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br;
- R²: is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br or an aromatic or heteroaromatic 6-membered ring, optionally substituted with at least one substituent selected from the group consisting of -OH, -O-C₁₋₆-alkyl, - C₁₋₆-alkyl, -O-benzyl, phenyl, F, Cl, and Br:
or R² is A-R⁴ wherein
R⁴ is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br;
A is O or NR⁵ wherein
R⁵ is independently hydrogen or a linear, branched or cyclic, saturated or unsaturated C₁₋₆-aliphatic residue, optionally substituted with at least one substituent selected from the group consisting of oxo (=O), -OH, - O-C₁₋₆-alkyl, -C₁₋₆-alkyl, -O-benzyl, phenyl, F, Cl, and Br;
- R³: is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br, or an aromatic or heteroaromatic 6-membered ring, optionally substituted with at least one substituent selected from the group consisting of -OH, -O-C₁₋₆-alkyl, - C₁₋₆-alkyl, -O-benzyl, phenyl, F, Cl, and Br;
- X: is O or NR⁶ wherein
R⁶ is independently as defined above for R⁵;
- Q: is O or NR⁷ wherein
R⁷ is independently as defined above for R⁵; and
wherein each of the phenyl rings in formula (I) can have one or more further substituents, preferably selected from OH, NH₂, NHR⁸ wherein R⁸ is independently as defined above for R⁵, NR⁹R¹⁰ wherein R⁹,R¹⁰ are independently as defined above for R⁵; alkyl such as C₁₋₆-alkyl, -O-alkyl such as -O-C₁₋₆-alkyl, alkenyl such as C₂₋₆-alkenyl, -O-alkenyl such as -O-C₂₋₆-alkenyl, cycloalkyl such as C₄₋₆-cycloalkyl, heterocycloalkyl such as 4- to 6-membered heterocycloalkyl, aralkyl such as benzyl, -O-aralkyl such as-O-benzyl, aryl such as phenyl, heteroaryl such as 4- to 6-membered heteroaryl, halo such as F, Cl, Br, -CN, -SO₃H, -COOH and corresponding esters such as C₁₋₆-alkyl esters and alkyl amides such as C₁₋₆-alkyl amides, sulfonamides, and O- or -C-glycosidic bound sugars;
optionally, in the form of the corresponding pharmaceutically acceptable salts and/or solvates thereof.

In case 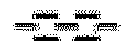 in the above formula (I) is a double bond, the stilbenoid compound is preferably a trans-stilbene, i,e, the double bond is a trans-configured double bond.

In preferred compounds of formula (I) R¹ is hydrogen or C₁₋₃-alkyl. In further preferred embodiments of the invention, R² is hydroxyl or a lower alkoxy group, preferably C₁₋₆-alkoxy, more preferably methoxy or ethoxy. Further preferred compounds of formula (I) are compounds wherein R³ is a lower alkyl group, in particular C₁₋₆-alkyl, more preferably methyl or ethyl. In further preferred compounds of the above formula (I) X is O. In still further preferred compounds of the above formula (I) Q is O.

Particularly preferred compounds for use in the present invention are those in which in formula (I)
- R¹: is hydrogen or C₁₋₃-alkyl;
- R²: is selected from hydroxy, methoxy and ethoxy;
- R³: is C₁₋₆-alkyl, more preferably methyl or ethyl;
- X: is O; and
- Q: is O.

Examples of highly preferred compounds for use in the invention have the following structures:

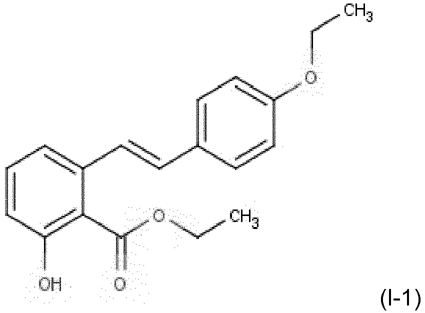

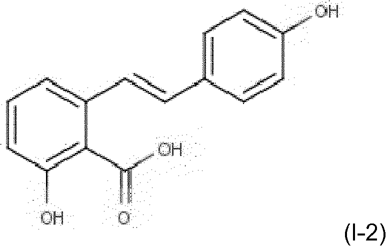

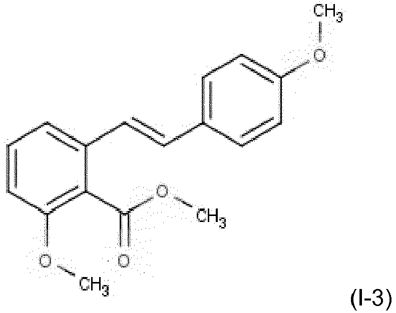

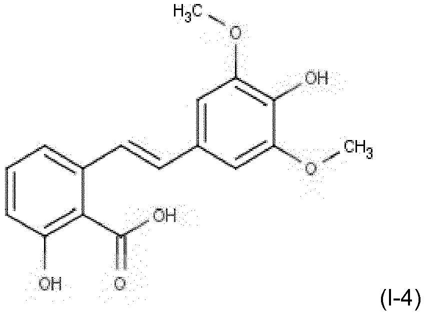

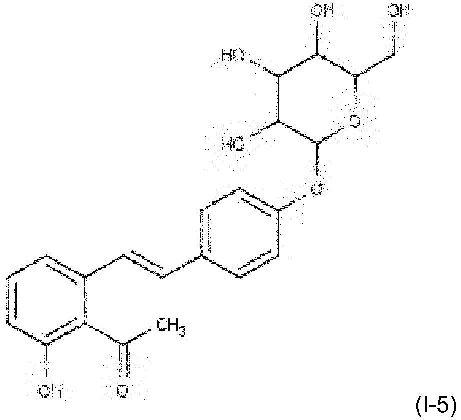

Most preferred, the compound of structure (I-1) is used in the embodiments of the invention.

It is to be understood that all the above compounds include their pharmaceutically acceptable salts and/or solvates, preferably hydrates, of these compounds.

Compounds for use in the present invention having structures as defined above may be prepared by synthesis starting from hydrangenol 8-β-D-glucoside which may be prepared from leaves of *Hydrange macrophylla* as disclosed in Hashimoto et al., *supra,* and according to synthetic principles described therein and general synthesis routes known to the skilled person.

According to the invention, it has been surprisingly found that the compounds of formula (I) are potent agonists of sirtuins, in particular sirtuins 1, 2 and/or 3, significantly enhance AMPK phosphorylation, and/or significantly increase the number of mitochondria in cells, preferably in liver cells and/or neurotrophic cells and/or adipocytes, more preferably in human liver cells and/or human neurotrophic cells and/or adipocytes. In particular, the compounds for use in the invention show a significantly greater efficacy, e.g. a higher sirtuin activation, and an improved dose-response behavior in comparison to trans-resveratrol which is described in the prior art as the most widely used sirtuin activator (see, e.g., Baur (2010), *supra*).

Thus, according to one embodiment, the present invention provides the use of the compounds of formula (I) in activating sirtuins and/or enhancing AMPK phosphorylation and/or increasing the number of mitochondria in cells, preferably liver cells and/or neurotrophic cells and/or adipocytes. Methods for such use may be carried out *in vitro,* e.g. using cell culture techniques known in the art.

The above characteristics (or at least one of them) make the compounds of formula (I) particularly suitable for medical applications, preferably as pharmaceutical actives for the treatment and/or prevention of a variety of diseases such as metabolic diseases, e.g. diabetes, lipid metabolic disorders, metabolic syndrome and obesity, and neurodegenerative diseases, e.g. Alzheimer's disease, progressive supranuclear palsy, corticobasal degeneration, agyrophilic grain disease, frontotemporal dementia and parkinsonism of chromosome 17, Pick's Disease, Parkinson's disease, Lewy bodies dementia, multisystem atrophy, frontotemporal lobar degeneration, neuronal intermediate filament inclusion disease, basophilic inclusion body disease, Huntington's disease, spinobulbar muscle atrophy type Kennedy, Friedreich ataxia, dentatorubral-pallidoluysian atrophy, spino-cerebral ataxia, Creutzfeld-Jakob disease, Germann-Sträussler-Scheinker syndrome, fatal familiar insomnia, Kuru, primary lateral Sclerosis, spinal muscle atrophy, amyotrophic lateral sclerosis, Seitelberger's disease, neurodegeneration with brain iron accumulation, frontotemporal lobar degeneration with ubiquitin system and familiar encephalopathy with neuroserpin inclusions.

The invention therefore further relates to pharmaceutical compositions containing a compound as defined above together with at least one pharmaceutically acceptable carrier, excipient and/or diluent.

A "pharmaceutical composition" as defined herein is a composition comprising a substance according to formula (I) typically in a basic pharmaceutical formulation, preferably a formulation such that the composition is suitable for systemic application, more preferably to humans, most preferably for oral application to humans.

In a preferred embodiment, the composition comprises a compound of general formula (I), e.g. a compound according to formula (I-1), (I-2), (I-3), (I-4) or (I-5), in an amount of from 0.0001 to 90 wt.-%, such as 0.001 to 50 wt.-%, or from 0.01 to 20 wt.-%, or from 0.1 to 10 wt.-% based on the total weight of the composition.

In one embodiment, the pharmaceutical composition according to the invention comprises at least one further active ingredient suitable for prevention and/or treatment of one or more of the conditions as outlined above. For example, the composition may comprise at least one further sirtuin activating and/or AMPK activating and/or compound increasing the number of mitochondria in cells. Examples of sirtuin activating compounds are disclosed, e.g. in WOA-2005/002672, WO-A-2006/079021, and WO-A-2009/061453.

According to preferred embodiments, the pharmaceutical composition further contains one or more antioxidants which may be selected from the group consisting of ascorbic acid and derivatives thereof such as erythorbic acid and sodium ascorbate, thiol derivatines such as thioglycerol, cysteine, acetylcysteine, cystine, dithioerythreitol, dithiothreitol and glutathione, tocopherols such as alpha-, delta- or gamma-tocopherol" BHA, BHT, sulfurous acid salts such as sodium sulfate, sodium sulfite, sodium bisulfite, acetone sodium bisulfite, sodium metabisulfite, sodium formaldehyde sulfoxylate, sodium thiosulfate, nordiydroguaiaretic acid and popyl gallate as well combinations thereof.

In a preferred embodiment, the total amount of antioxidants in the compositions of the invention is preferably 0.01 to 20 wt.-%, particularly preferably 0.05 to 10 wt.-%, in particular 0.1 to 5 wt.-%, based on the total weight of the composition.

In further embodiments, the compositions as defined herein contain a preservative as a further ingredient, which may preferably be selected from the group consisting of phenol and derivatives thereof such as cresol, parabenes (e.g. methyl-4-hydroxybenzoate) and chlorocresol, aliphatic and aromatic alcohols such as ethanol, propylene glycol, chlorobutanol and benyzlalcohol, organic quicksilver compounds such as thiomersal, quarternary ammonium compounds such as benzalconium chloride and cetyl pyridinium chloride, carboxylic acids such as sorbic acid and benzoic acid and its esters and salts as well as combinations of such preservatives.

A pharmaceutically acceptable carrier for use in the present invention is a pharmaceutically acceptable material, composition or vehicle, such as a liquid or solid, filler, diluent, excipient, solvent or encapsulating material, involved in carrying or transporting any compound or composition or component thereof as defined herein from one organ, or portion of the body, to another organ, or portion of the body. Each carrier must be "acceptable" in the sense of being compatible with the respective composition and its components and not injurious to the patient to be treated. Examples of materials which may serve as pharmaceutically acceptable carriers for use in the present invention include, but are not limited to, sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as coconut butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol, and polyethylene glycol; esters, such as ethyl oleate and ethyl laurate; agar; buffering agents, such as magnesium hydroxide and aluminum hydroxide; alginic acid; pyrogen-free water; isotonic saline; Ringer lactate; ethyl alcohol; phosphate buffer solutions; and other non-toxic compatible substances typically employed in pharmaceutical formulations.

In a preferred embodiment, the compositions according to the invention comprise a total amount of 5 to 70 wt.-%, preferably 7.5 to 60 wt.-%, more preferably 10 to 50 wt.-%, even more preferably 10 to 40 wt.-%, of one or more pharmaceutically acceptable carriers as defined above, in each case based on the total weight of the composition.

In one embodiment, the compositions according to the invention comprise water, preferably pyrogen-free water, in an amount of up to 98 wt.-%, preferably 10 to 95 wt.-%, more preferably 25 to 90 wt.-%, in each case based on the total weight of the composition.

Pharmaceutical composition according to the invention may take the form of tablets, lozages, capsules, solutions for injection or infusion, suppositories, sprays, flushings and the like.

As already noted above, the pharmaceutical compositions according to the invention are particularly intended for systemic administration such that the active ingredient(s), in particular a compound of formula (I), enter(s) the patient's system, and, thus, is subject to metabolism and other like processes. Systemic administration includes oral, transdermal, buccal and parenteral administration as well as inhalation and insufflation. Parenteral administration is typically by injection and includes, but is not limited to, intravenous, intramuscular, intraaterial, intrathecal, intracapsular, intraorbital, intracardiac, intradermal, intreperitoneal, transtracheal, subcutaneous, subcuticular, intra-articular, subcapsular, subarachnoid, intraspinal, and intrasternal injection and/or infusion.

Pharmaceutical compositions according to the invention are typically formulated in a conventional manner, and general guidance in this regard as well as applicable routes of administration may be taken from, e.g. Loyd V. Allen Jr. (ed.): Remington: The Science and Practice of Pharmacy, 22nd ed., Pharmaceutical Press, London, 2012, or L. Brunton, B.A. Chalner, B. Knollman (eds.): Goodman and Gilman's The Pharmacologic Basis of Therapeutics, 12th ed. McGraw-Hill, New York, 2011.

For injection or infusion, the compounds for use as defined herein are typically formulated in liquid solutions, preferably in physiologically compatible buffers such as Hank's solution or Ringer Lactate. In addition, the compounds of formula (I) may be formulated in solid form, e.g. a lyophilized form, and redissolved or suspended shortly or immediately prior to use. Formulations for injection are typically presented in unit dosage form, e.g. in ampoules, vials or multi-dose containers, preferably with an added preservative (typically as outlined above). The compositions for injection may be in the form of suspensions, solutions or emulsions, and may contain additional formulating agents such as suspending, stabilizing, emulsifying and/or dispersing agents.

For oral administration, the pharmaceutical composition may be prepared in the form of a tablet or other entity suitable for oral application (as already outlined above) by conventional means with pharmaceutically acceptable excipients such as binding agents (e.g. pregelatinised maize starch, polyvinylpyrrolidone or hydroxypropyl methylcellulose), fillers (e.g. lactose, microcrystalline cellulose or calcium hydrogen phosphate), lubricants (e.g. magnesium stearate, talc or silica), disintegrants (e.g. potato starch or sodium starch glycolate) or wetting agents (e.g. sodium lauryl sulphate). Formulations for oral administration such as tablets may be provided with coatings, as desired or needed. Formulations for oral administration may also be in liquid form such as solutions, syrups or suspensions. Alternatively, the composition may be in dry form for reconstitution with a suitable vehicle, e.g. water, before use. Liquid formulations typically contain pharmaceutically acceptable additives such as suspending agents (e.g. sorbitol syrup, cellulose derivatives or hydrogenated edible fats), emulsifiers (e.g. lecithin or acacia), non-aqueous vehicles (e.g. oils, oily esters, ethanol or fractionated vegetable oils) and preservatives (as already outlined above). Oral preparations may also include further ingredients such as buffer salts, flavoring, coloring and sweetening agents as appropriate. Pharmaceutical compositions of the invention may also be formulated for controlled release of the active agent(s), in particular a compound of formula (I).

For inhalation, the pharmaceutical composition may take the form of an aerosol spray delivered from a pressurized pack or nebulizer by the use of a suitable propellant such as dichlorodifluoromethane, trichlorofluoromethane, dichlorotetrafluoroethane, carbon dioxide or other suitable gas.

Pharmaceutical compositions of the invention can also be formulated for rectal administration, and are typically in the form of suppositories or retention enemas containing conventional suppository bases such as cacao butter or other glycerides.

Compositions of the invention may also be formulated as depot preparations for long term delivery of the active compound(s). Such long term administration can be carried out, e.g. by, preferably subcutaneous or intramuscular, implantation or by intramuscular injection. For this type of administration, the compounds for use in the present invention may be formulated with suitable polymeric and/or hydrophobic materials (e.g. as an emulsion in a suitable oil) or ion exchange resins, or as sparingly soluble derivatives such as sparingly soluble salts of the compounds as described herein.

Transdermal administration includes corresponding compositions applied in the form of transdermal patches which may be embodied, e.g. as single-layer drug-in-adhesive form, multi-layer drug-in-adhesive form, reservoir form, matrix form or as a vapour patch. For transdermal formulation the composition of the invention typically contains one or more skin permeation enhancer(s) serving to facilitate passage of therapeutic levels of the active compound(s) to pass through a reasonably sized area of the skin. Suitable enhancers are well known in the art and include, e.g. lower alcohols such as methanol, thanol and 2-propanol, alkyl methyl sulfoxides such as dimethylsulfoxide, decylmethylsulfoxide and tetradecylmethylsulfoxide, pyrrolidones such as 2-pyrrolidone, N-methyl-2-pyrrolidone and N-(hydroxyethyl)pyrrolidone, urea, N,N-diethyl-m-toluamide, C₂₋₆-alkanediols, miscellaneous solvents such as dimethyl formamide, N,N-dimethylacetamide and tetrahydrofuryl alcohol, and 1-substituted azacycloheptan-2-ones such as 1-n-dodecylcyclazacycloheptan-2-one (laurocapram).

The present invention further relates to a method for the prevention and/or treatment of a condition selected from the group consisting of metabolic disorders and neurodegenerative diseases comprising the step of administering an effective amount of a pharmaceutical composition to a subject, such as an animal, preferably a human, in need of such treatment. Preferred conditions that may be treated by the compounds of formula (I) have already been elaborated above..

A "therapeutically effective" amount of the pharmaceutical composition means an amount of the composition that exerts the desired effect in the subject for preventing and/or treating the condition. It is influenced by several factors such as weight, age, sex and condition of the subject as well as the formulation and route of administration. The "therapeutically effective" amount of the composition is usually be determined by conventional experimentation, e.g. using suitable cell culture and/or animal models.

The following non-limiting examples further illustrate the present invention.

### EXAMPLES

### Example 1: Agonistic effect on sirtuins

The agonistic effect of compounds of formula (I) on human sirtuin-1 (Sirt1) was determined *in vitro* and compared with the known agonistic effect of trans-resveratrol as benchmark.

Compounds were tested for Sirt1 activation using the SIRT1 Fluorimetric Drug Discovery Kit (BioMol, Hamburg, Germany), Testing was carried out in duplicate following the manufacturer's instructions. Assay medium: 50mM Tris-HCl pH 8.0, 137 mM NaCl, 2.7 mM KCI, 1 mM MgCl₂, 1 mg/ml bovine serum albumin (BSA). Stock solutions of the test compounds (10 mM in DMSO) were used, yielding a final concentration of 10 µM in the assay plate. Readout was obtained by measuring the fluorescence in a microtiter plate reader (excitation: 380 nm; detection: 440 nm).

### (a) Screening at 10 µM concentration: results are shown in Table 1

**Table 1: Agonistic effect on Sirt1 in comparison to trans-resveratrol**

| **Compound** | **Fold increase in sirt1 activity versus control** |
|---|---|
| (I-1) | 23.9 |
| (I-2) | 4.8 |
| (I-3) | 1.1 |
| (I-4) | 2.0 |
| (I-5) | 2.7 |
| trans-resveratrol (control) | 1.0 |

### (b) Quantification of the Sirt1 agonistic effect, dose response

The agonistic effect on Sirt1 was determined at different concentrations. For this purpose, the test compound stock solutions were diluted to yield concentrations of 0.3, 1, 3, 10 and 30 µM, respectively, in the assay, which was performed as described above. The compound of structure (I-1) was selected for this experiment. The dose-response behaviour observed was found clearly superior in comparison to trans-resveratrol (Table 2).

**Table 2: Dose-response effect of compound (I-1) on Sirt1 in comparison to trans-resveratrol**

| **Concentration [µM]** | **Activity [% control]** | |
|---|---|---|
| | compound (I-1) | trans-resveratrol |
| 30.0 | 1381 | 120 |
| 10.0 | 626 | 108 |
| 3.0 | 434 | 100 |
| 1.0 | 233 | 101 |
| 0.3 | 147 | 104 |

Quantification using the GraphPad Prism 5.0 software (GraphPad Software, Inc., LaJolla, CA, USA) led to the following EC300 values (i.e. the concentration that gave 3-times the basal response of the enzyme = activation by a factor of three):
Compound of formula (I-1): EC300 = 2.0 µM
Trans-resveratrol: EC300 > 30 µM

### Example 2: Selectivity of agonistic effect on sirtuins

The selectivity of the agonistic effect of the compound of formula (I-1) on different sirtuin isoforms was investigated. For this purpose, *in vitro* testing was performed as described in Example 1 using the respective SIRT1 Fluorimetric Drug Discovery Kit, SIRT2 Fluorimetric Drug Discovery Kit, and SIRT3 Fluorimetric Drug Discovery Kit (each from BioMol, Hamburg, Germany). The results demonstrate that compound (I-1) has an agonistic activity on all three sirtuin isoform and shows similar dose-response behaviours on Sirt1 and Sirt2. The dose-response effect is slightly lesser on Sirt3.

**Table 3: Activity on Sirt1**

| **Concentration [µM]** | **Activity [% control]** | |
|---|---|---|
| | compound (I-1) | trans-resveratrol |
| 30.0 | 515 | 145 |
| 10.0 | 256 | 111 |
| 3.0 | 168 | 117 |
| 1.0 | 121 | 100 |
| 0.3 | 111 | 121 |

**Table 4: Activity on Sirt2**

| **Concentration [µM]** | **Activity [% control]** | |
|---|---|---|
| | compound (I-1) | trans-resveratrol |
| 30.0 | 914 | 105 |
| 10.0 | 530 | 100 |
| 3.0 | 204 | 97 |
| 1.0 | 142 | 104 |
| 0.3 | 114 | 104 |

**Table 5: Activity on Sirt3**

| **Concentration [µM]** | **Activity [% control]** | |
|---|---|---|
| | compound (I-1) | trans-resveratrol |
| 30.0 | 247 | 132 |
| 10.0 | 216 | 113 |
| 3.0 | 156 | 116 |
| 1.0 | 124 | 114 |
| 0.3 | 109 | 105 |

Quantification using the GraphPad Prism 5.0 software (GraphPad Software, Inc., LaJolla, CA, USA) leads to the following EC300 values (Table 6):

**Table 6: EC300 values of compound (I-1) with respect to sirtuin isoforms**

| **Sirtuin isoform** | **EC300 compound (I-1) [µM]** | **EC300 trans-resveratrol [µM]** |
|---|---|---|
| Sirt1 | 12.9 | >30 |
| Sirt2 | 4.9 | >30 |
| Sirt3 | >30* | >30 |

| | | |
|---|---|---|
| * However: clear activation in dose-response curve visible. | | |

### Example 3: Effect of compound (I-1) on AMPK

The effect of compound (I-1) on 5'-AMP-activated protein kinase (AMPK) was measured in normal human epidermal keratinocytes (NHEK). AMPK is activated by phosphorylation as phospho-AMPK (P-AMPK). The degree of phosphorylation was evaluated by Western blot. Glycerinaldehyde-3-phosphate dehydrogenase (GAPDH) was used as housekeeping protein and loading control. The level of AMPK phosphorylation was determined by calculating the P-AMPK/GAPDH ratio. 5-Amino-1-ß-D-ribofuranosyl-imidazole-4-carboxamide (AICAR), a known stimulator of AMPK activation (Corton et al. (1995), Eur. J. Biochem. 229, pp. 558-565), was used as positive control.

Culture conditions: 37 °C, 5 % CO₂. Culture medium: Keratinocyte-SFM (serum-free medium) containing human recombinant epidermal growth factor (EGF 1-53) 0.25 ng/mL and bovine pituitary extract 25 µg/mL (Gibco; Cat. No. 17005-075), supplemented with gentamycin 25 µg/mL. Assay medium: Keratinocyte-SFM (Gibco; Cat. No. 10744-019), supplemented with gentamycin 25 µg/mL.

NHEK were seeded in 24-well plates (30.000 cells / well) and cultured in culture medium for 24 h and then in assay medium for another 24 h. Medium was then removed and replaced by assay medium containing the respective test compound or reference in defined concentrations.

Cytotoxicity was determined using the 3-(4,5-dimethylthiazol-2-yl)-2,5-diphenyltetrazoliumbromid (MTT) reduction assay (sse T. Mosmann (1983) J. Immunol. Methods. 65, pp. 55-63). All cells were found to be fully viable except for those incubated with trans-resveratrol at 100 µM (2 % viability) and at 300 µM (43 % viability), illustrating the cytotoxic effect of trans-resveratrol on NHEK at the respective concentrations. At the same concentrations, NHEK cells with compound (I-1) retained full viability.

### Western blot analysis of AMPK phosphorylation

The cells were incubated for 48 h in assay medium containing the test items. Cell lysis was performed using Cell Extraction Buffer (Invitrogen, Cat. No. FNN0011), supplemented with the Phosphatase Inhibitor Cocktail 1 (Sigma-Aldrich, Cat. No. P2850). The protein amount was quantified using the DC™ protein assay (Biorad, Cat. No. 500-0112). Volumes were adjusted in order to load for each condition 20 µg of total proteins per lane. Proteins were denaturized in Laemmli buffer (Biorad, Cat. No. 161-0737) at 95 °C, subjected to electrophoresis on a 10 % polyacrylamide gel, and then transferred onto nitrocellulose membrane. After saturation in phosphate buffered saline (PBS)/Tween/bovine serum albumin (BSA), P-AMPK and GAPDH were successively detected using specific primary antibodies (Anti-P-AMPK, ref #4188, Cell signalling technology; Anti-GAPDH [HRP], ref #ab9482, ABCAM). P-AMPK primary antibody was detected using an appropriate horseradish peroxidise (HRP)-conjugated secondary antibody whereas GAPDH was directly conjugated to HRP. A single blot was developed successively for each of the markers. After each antibody incubation the blot was washed using stripping buffer allowing for fixation of the next antibody. The different markers were developed by enhanced chemiluminescence (ECL Plus Amersham™, GE Healthcare). Image acquisition was performed with a scanner of chemiluminescence (FUJI LAS 3000, Fujifilm); and densitometric analysis was obtained using Multigauge software (Fujifilm).

**Table 7: Experiment 1**

| | Conc. [µM] | P-AMPK [AU/mm²] | GAPDH [AU/mm²] | Ratio P-AMPK/ GAPDH | AMPK phosphorylation [% Control] |
|---|---|---|---|---|---|
| *Membrane 1* | | | | | |
| Control | -- | 196 | 463 | 0.42 | 100 |
| AICAR | 1000 | 1060 | 518 | 2.05 | 482 |
| Compound (I-1) | 3 | 123 | 480 | 0.26 | 60 |
| | 10 | 202 | 528 | 0.38 | 90 |
| | 30 | 2554 | 599 | 4.26 | 1006 |

| *Membrane 2* | | | | | |
|---|---|---|---|---|---|
| Control | -- | 296 | 373 | 0.79 | 100 |
| trans-resveratrol | 300 | 1616 | 282 | 5.73 | 722 |

| | | | | | |
|---|---|---|---|---|---|
| AU = arbitrary units | | | | | |

**Table 8: Experiment 2**

| | Conc. [µM] | P-AMPK [AU/mm²] | GAPDH [AU/mm²] | Ratio P-AMPK/GAPDH | AMPK phosphorylation [% Control] |
|---|---|---|---|---|---|
| Control | -- | 249656 | 2208428 | 0.113 | 100 |
| DMSO | 2% | 306555 | 1978162 | 0.155 | 137 |
| AICAR | 1000 | 1377449 | 2406346 | 0.572 | 506 |
| trans-resveratrol | 10 | 228626 | 2511753 | 0.091 | 81 |
| | 30 | 211794 | 2177595 | 0.097 | 86 |
| | 100 | 327757 | 2479461 | 0.132 | 117 |
| | 200 | 2361575 | 2561347 | 0.922 | 816 |
| | 300 | 69290 | 2912376 | 0.024 | 21 |

| | | | | | |
|---|---|---|---|---|---|
| AU = arbitrary units | | | | | |

### Conclusions:

i) Compound (I-1) significantly enhanced AMPK phosphorylation at 30 µM. Surprisingly, trans-resveratrol needed about 10 times higher dosage (at least 200 µM) for a similar effect. Such concentrations (100 µM trans-resveratrol and higher) already had cytotoxic effects on NHEK under assay conditions.
ii) The effect of compound (I-1) is significantly higher than that of the reference AICAR.
iii) Phosphorylation of AMPK in NHEK in presence of trans-resveratrol is observed for such concentrations only which showed cytotoxic effects, whereas no cytotoxicity against NHEK was observed for compound (I-1) at all tested concentrations.

### Example 4: Effect of compound (I-1) on mitochondria biosynthesis

The effect of compound (I-1) on mitochondria biosynthesis was investigated in normal human dermal fibroblasts (NHDF). NHDF were obtained from PromoCell (Lot No. 006151; female Caucasian donor; age 61 years; from eyelid skin) and cultivated in Fibroblast Growth Medium (PromoCell; Cat. No. C-023020) supplemented with Supplement Mix (PromoCell; Cat. No. C-039215) at 37 °C /5% CO₂. NHDF were seeded in flat-bottom 96-well plates (5000 cells / well) and incubated in said medium for 24 h or 48 h, respectively, with the compounds present in concentrations ranging from 0.03 µM to 200 µM. Mitochondria content was analyzed using the Mito-ID® Green Detection Kit for fluorescence microscopy (Enzo Life Sciences; Cat. No. ENZ-51055-K500). Fluorometric analysis was done using a Spectramax plate reader (Molecular Devices) and following the manufacturer's protocol. Cytotoxicity was determined in parallel by fluorometric readout using the Resazurin Based *In Vitro* Toxicology Assay Kit (Sigma Aldrich, Cat. No. TOX-8), and following the manufacturer's protocol.

### Results:

The total content of mitochondria in NHDF was significantly enhanced by compound (I-1) after incubation for 24 h and 48 h, respectively, while no cytotoxicity or loss of cell viability was observed. Significant enhancement of mitochondria biosynthesis was observed at concentrations of about 10 µM or higher.

**Table 9: Mitochondria content of NHDF after cultivation with compound (I-1)**

| | 24 h incubation time | | 48 h incubation time | |
|---|---|---|---|---|
| Conc. [µM] | Signal intensity [% Control] | Standard deviation | Signal intensity [% Control] | Standard deviation |
| 0.03 | 100 | 1.8 | 97 | 5.9 |
| 0.09 | 99 | 1.3 | 92 | 0.4 |
| 0.27 | 100 | 2.3 | 91 | 1.2 |
| 0.82 | 101 | 1.5 | 93 | 1.3 |
| 2.47 | 103 | 3.1 | 94 | 1.3 |
| 7.41 | 114 | 3.8 | 103 | 2.3 |
| 22.22 | 182 | 21.7 | 147 | 7.1 |
| 66.67 | 354 | 62.0 | 293 | 23.5 |
| 200.00 | 906 | 64.4 | 765 | 62.5 |

## Claims

1. A compound of general formula (I) for use as a medicament in systemic application in humans
wherein 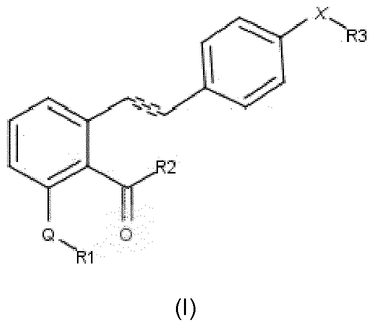
is a single or double bond
R¹ is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br;
R² is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br or an aromatic or heteroaromatic 6-membered ring, optionally substituted with at least one substituent selected from the group consisting of -OH, -O-C₁₋₆-alkyl, -C₁₋₆-alkyl, -O-benzyl, phenyl, F, Cl, and Br: or is A-R⁴ wherein
R⁴ is independently hydrogen or a linear, branched or cyclic, saturated or unsaturated C₁₋₆-aliphatic residue, optionally substituted with at least one substituent selected from the group consisting of oxo (=O), -OH, - O-C₁₋₆-alkyl, -C₁₋₆-alkyl, -O-benzyl, phenyl, F, Cl, and Br;
A is O or NR⁵ wherein
R⁵ is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br;
R³ is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br, or an aromatic or heteroaromatic 6-membered ring, optionally substituted with at least one substituent selected from the group consisting of -OH, -O-C₁₋₆-alkyl, -C₁₋₆-alkyl, -O-benzyl, phenyl, F, Cl, and Br;
X is O or NR⁶ wherein
R⁶ is independently as defined above for R⁵;
Q is O or NR⁷ wherein
R⁷ is independently as defined above for R⁵; and
wherein each of the phenyl rings in the formula (I) can have one or more further substituents selected from OH, NH₂, NHR⁸ wherein R⁸ is independently as defined above for R⁵, NR⁹R¹⁰ wherein R⁹,R¹⁰ are independently as defined above for R⁵; -O-C₁₋₆-alkyl, -C₁₋₆-alkyl, -O-C₂₋₆-alkenyl, C₂₋₆-alkenyl, C₄₋₆-cycloalkyl, 4- to 6-membered heterocycloalkyl, benzyl, -O-benzyl, phenyl, 4 to 6-membered heteroaryl, F, Cl, Br, - CN, -SO₃H, -COOH and corresponding C₁₋₆-alkyl esters and C₁₋₆-alkyl amides, sulfonamides, and O- or -C-glycosidic bound sugars;
optionally, in the form of the corresponding pharmacologically acceptable salts and/or solvates thereof.

2. The compound for use of claim 1 wherein
R¹ is hydrogen or C₁₋₃-alkyl;
R² is C₁₋₆-alkyl or O-C₁₋₆-alkyl;
X is O; and
Q is O; and
R³ is C₁₋₆-alkyl.

3. The compound for use of claim 1 or 2 wherein 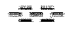 is a trans-configurated double bond.

4. The compound for use according to any one of the preceding claims wherein the compound of formula (I) has the following structure:

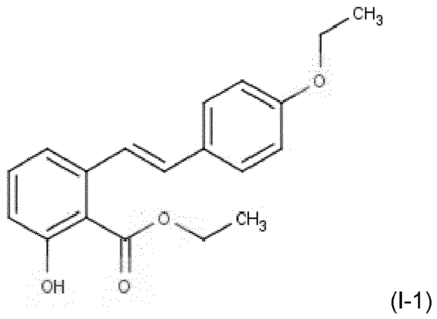

5. The compound as defined in any one of the preceding claims for use in the prevention and/or treatment of a condition selected from the group consisting of metabolic disorders and neurodegenerative diseases.

6. The compound for use of claim 5 wherein the metabolic disorder is selected from the group consisting of diabetes, insulin resistance, lipid metabolic disorders, metabolic syndrome and obesity.

7. The compound for use of claim 5 wherein the neurodegenerative disease is selected from the group consisting of Alzheimer's disease, Parkinson's disease, Huntington's disease, Lewy bodies dementia, and amyotrophic lateral sclerosis.

8. Use of the compound as defined in any one of claims 1 to 4 for activating sirtuins and/or enhancing AMPK phosphorylation and/or increasing the number of mitochondria in cells, preferably liver cells and /or neurotrophic cells and / or adipocytes.

9. A pharmaceutical composition comprising a compound as defined in any one of claims 1 to 4 together with at least one pharmaceutically acceptable carrier, excipient and/or diluent.

10. A method for the prevention and/or treatment of a condition selected from the group consisting of metabolic disorders and neurodegenerative diseases comprising the step of administering an effective amount of a composition of claim 9 to a subject in need of such treatment.

11. The method of claim 10 wherein the metabolic disorder is selected from the group consisting of diabetes, insulin resistance, lipid metabolic disorders, metabolic syndrome and obesity.

12. The method of claim 10 wherein the neurodegenerative disease is selected from the group consisting of Alzheimer's disease, Parkinson's disease, Huntington's disease, Lewy bodies dementia, and amyotrophic lateral sclerosis.
